# EUROPEAN PATENT SPECIFICATION

(11) **EP 1 509 621 B1**
(45) Date of publication and mention of the grant of the patent: **09.09.2009**
(21) Application number: 03740698.0
(22) Date of filing: 30.05.2003
(51) Int. Cl.: C12Q 1/68, G01N 33/68, G01N 33/74

(54) **DIAGNOSTIC OF ALZHEIMER'S DISEASE BY ANALYSIS OF IL-10 GENE POLYMORPHISMS**
DIAGNOSE DER ALZHEIMER ERKRANKUNG DURCH ANALYSE von IL-10 GEN POLYMORPHISMEN
DIAGNOSTIC DE LA MALADIE D'ALZHEIMER PAR ANALYSE DE POLYMORPHISMES DU GENE IL-10

(30) Priority: 31.05.2002 GB 0212648
(43) Date of publication of application: 02.03.2005
(73) Proprietor: Immunoclin Limited, London N12 8NP (GB)
(72) Inventor: CLERICI, Mario Milano University Medical School, I-20157 Milano (IT); ANNONI, Giorgio Department of Internal Medicine, I-20122-Milan (IT)
(74) Representative: McQueen, Andrew Peter
(86) International application number: PCT/GB2003/002369
(87) International publication number: WO 2003/102237

(56) References cited:
- WO-A-95/33992
- LIO D ET AL: "Interleukin-10 promoter polymorphism in sporadic Alzheimer's disease." GENES AND IMMUNITY, vol. 4, no. 3, April 2003 (2003-04), pages 234-238, XP001155652 ISSN: 1466-4879 (ISSN print)
- REMARQUE E J ET AL: "Patients with Alzheimer's disease display a pro-inflammatory phenotype" EXPERIMENTAL GERONTOLOGY, vol. 36, no. 1, January 2001 (2001-01), pages 171-176, XP002257920 ISSN: 0531-5565
- TURNER D M ET AL: "AN INVESTIGATION OF POLYMORPHISM IN THE INTERLEUKIN-10 GENE PROMOTER" EUROPEAN JOURNAL OF IMMUNOGENETICS, OXFORD, GB, vol. 24, no. 1, 1997, pages 1-8, XP000865679 ISSN: 0960-7420
- PAPASSOTIROPOULOS ANDREAS ET AL: "Genetics of interleukin 6: Implications for Alzheimer's disease" NEUROBIOLOGY OF AGING, vol. 22, no. 6, November 2001 (2001-11), pages 863-871, XP002257921 ISSN: 0197-4580
- MARTIN E R ET AL: "SNPING AWAY AT COMPLEX DISEASES: ANALYSIS OF SINGLE-NUCLEOTIDE POLYMORPHISMS AROUND APOE IN ALZHEIMER DISEASE" AMERICAN JOURNAL OF HUMAN GENETICS, AMERICAN SOCIETY OF HUMAN GENETICS, CHICAGO, IL, US, vol. 67, August 2000 (2000-08), pages 383-394, XP000995224 ISSN: 0002-9297
- DEPBOYLU CANDAN ET AL: "Lack of association of interleukin-10 promoter region polymorphisms with Alzheimer's disease." NEUROSCIENCE LETTERS, vol. 342, no. 1-2, 15 May 2003 (2003-05-15), pages 132-134, XP001155649 ISSN: 0304-3940 (ISSN print)
- SZCZEPANIK A M ET AL: "IL-4, IL-10 and IL-13 modulate ab(1-42)-induced cytokine and chemokine production in primary murine microglia and a human monocyte cell line" JOURNAL OF NEUROIMMUNOLOGY, ELSEVIER SCIENCE PUBLISHERS BV, XX, vol. 113, no. 1, 2001, pages 49-62, XP002955731 ISSN: 0165-5728

## Description

This invention relates to the use of IL-10 in the diagnosis, of Alzheimer disease.

The major cause of cognitive decline in the elderly is Alzheimer's disease (AD). Because of longer life spans worldwide, the number of people that will be affected by AD is expected to triple over the next 50 years (1). AD is a clinical syndrome characterised by complex and heterogeneous pathogenic mechanisms. The recognised genetic factors include mutations of the gene encoding the amyloid precursor protein (2), presenilin 1 and 2 (3, 4), which account for a small part of familial and usually early-onset AD cases. Genetic factors have also been associated with the sporadic or non-familial form of the disease and the allele e4 of the apolipoprotein E (Apo E) significantly increases the risk of AD, but is neither necessary nor sufficient for the development of the disease (5- 7). Therefore other genetic and environmental factors are likely to be implicated and are actively investigated.

Molecules that take part in the inflammatory cascade are of great interest, because inflammation has repeatedly been suggested to be associated with the neurodegenerative process characteristic of the AD brain (8). Thus, reactive astrocytosis is observed both in the cortex and in the hippocampus of these patients and the glial cells are also activated within or near the neuritic plaques. Over-expression of cytokines and other inflammatory molecules are common features of the AD brain pathology (9). Additionally, epidemiological studies showed that the long term use of non-steroid anti-inflammatory drugs is associated with a decreased incidence of AD in a co-twin control study (10) and several other clinical studies confirmed a decreased association of AD in individuals treated with anti-inflammatory drugs (11) including COX2 specific inhibitors (12). These findings support the hypothesis that inflammation might contribute to the neurodegeneration associated with AD (13).

In the attempt to better understand the biology of AD the possible role of several cytokines and chemokines has recently been investigated. Virtually all of the mediators analyzed in these studies, including IL-1b, IL-6, TNF-α, IL-8, TGF-β and macrophage inflammatory protein-1a (MIP-1a), have been suggested to be up-regulated in AD compared to non demented controls (14-18). On the contrary, conflicting results are obtained in relation to the immunomodulatory cytokine IL-10, a type-2 cytokine that suppresses T lymphocytes and cell-mediated immunity in humans and mice and has potent anti-inflammatory properties (19- 21).

These studies considered each cytokine independently as gene polymorphisms and/or production, but never investigated the relationship between factors acting for and against inflammation, such as IL-10 and IL-6, in the same population sample.

It is worth recalling that single nucleotide polymorphisms (SNPs) in the promoter region of these two genes are known. The gene encoding IL-10, mapped to chromosome 1 between 1q31 and 1q32, is highly polymorphic. IL-10 production is correlated to biallelic polymorphisms at positions: -1082 (guanine to adenine substitution), -819 (thymine to cytosine substitution), and -592 (adenine to cytosine substitution). The polymorphism at position -1082 lies within an Ets (E-twenty-six specific)-like recognition site and may affect the binding of this transcriptional factor and therefore alter transcription activation; the -1082 A allele correlates with IL-10 generation after stimulation of T cells *in vitro* (57), while polymorphisms at positions -819 and -592 do not seem to be involved. The IL-6 gene in humans is organised in five exons and four introns and maps to the short arm of chromosome 7 (7p21) (50, 73). The involvement of IL-6 in many biological functions is paralleled by genetic associations of its allelic variants with several physiological and pathophysiological conditions. Two of its polymorphic sites have been frequently used for genetic association studies: a multiallelic variable number of tandem repeats (VNTR) polymorphism in the 3' flanking region (AT repeats) and a biallelic G-to-C polymorphism of the promoter at the position -174. The G/C single nucleotide polymorphism (SNP) seems to be associated with varying blood levels and transcription rates of IL-6 (54, 56, 68).

In the light of these considerations and on the basis of a case-control association study in Italian sporadic late-onset AD patients and matched healthy controls (HC), the present inventors evaluated whether IL-10 and IL-6 SNPs were related with the development of AD. The results shed further light on the inflammatory pathogenic hypothesis of AD and suggest an independent genetic predisposition from the metabolic one.

These allelic variations are associated with measurable differences in IL-10 and IL-6 production by antigen- and mitogen-stimulated peripheral blood lymphocytes. In fact, these polymorphisms occur in the regulatory region of the gene and are associated with high, intermediate or low IL-10 production (22).

The present inventors investigated beta amyloid-stimulated IL-10 and IL-6 production by peripheral blood lymphocytes (PBMC) of AD patients and of age-matched healthy controls. Because the generation of this cytokine was significantly reduced in AD patients, IL-10 polymorphisms were analysed in these same individuals. Results showed that the high IL-10-producing allele is extremely rare in AD patients.

Specifically, IL-10 genotypes are differently distributed when AD are compared with HC (χ² = 16.007; p=0.007). Therefore genotypes corresponding to reduced IL-10 production have a significantly higher distribution amongst AD subjects (table I). The presence of low-IL-10-producing genotypes is associated with a worsened clinical outcome of AD as follows: 1) earlier age at disease onset (Table II); and 2) faster disease progression (MMSE score)(Table III).

**Table I. IL-10 genotype distribution**

| | AD | HC | AD | HC |
|---|---|---|---|---|
| Genotype (c) | n=47 | n=25 | % | % |
| GCC/GCC (H) | 1 | 7 | 2 | 28 |
| GCC/ACC (M) | 10 | 9 | 21 | 36 |
| GCC/ATA (M) | 11 | 3 | 23 | 12 |
| ACC/ACC (L) | 8 | 1 | 17 | 4 |
| ACC/ATA (L) | 12 | 4 | 26 | 16 |
| ATA/ATA (L) | 5 | 1 | 11 | 4 |

The frequency of the different genotypes among Alzheimer's disease patients (AD) are statistically different from those of the healthy controls (HC). χ² = 16.007, df= 5, p= 0.007. In the brackets (c) there are the corresponding phenotype high (H), intermediate (M), low (L).

**Table II. IL-10 genotype distribution and age at onset**

| Genotype | mean | S.D. | SEM |
|---|---|---|---|
| | | | |
| GCC/GCC | 76 | / | / |
| GCC/ACC | 75.00 | 8.57 | 3.03 |
| GCC/ATA | 67.33 | 8.2 | 2.73 |
| ACC/ACC | 76.20 | 8.79 | 3.93 |
| ACC/ATA | 77.17 | 4.07 | 1.66 |
| ATA/ATA | 65.75 | 1.71 | 0.85 |

Correlation between the different genotypes in Alzheimer's disease patients and the age at onset. ANOVA: p= 0.042.

**Table III. IL-10 genotype distribution and MMSE**

| Genotype | mean | S.D. | SEM |
|---|---|---|---|
| | | | |
| GCC/GCC | 18 | | |
| GCC/ACC | 21.75 | 5.5 | 1.94 |
| GCC/ATA | 16.33 | 5.68 | 1.89 |
| ACC/ACC | 10.80 | 7.5 | 3.35 |
| ACC/ATA | 13.83 | 5.19 | 2.12 |
| ATA/ATA | 22.5 | 1.73 | 0.87 |

Correlation between the different genotypes in Alzheimer's disease patients and MMSE ANOVA: p= 0.010.

Chronic inflammation is suggested to be involved in the neurodegenerative process characteristic of AD (24, 25); this suggestion stems from both *in vivo* and *ex adjuvantibus* criteria. Hence, inflammatory mediators and activated glial cells are observed to be closely associated with neuritic plaques *in vivo.* Furthermore, recent data indicate that anti-inflammatory therapy could be useful in modulating disease progression (10-12). Despite this vast body of evidence, the biologic correlates of AD are still unclear. To shed light on this problem, attention was focused on IL-10. This cytokine is a pivotal regulatory cytokine involved in many facets of the immune response and is dysregulated in human autoimmune (26), malignant (27- 31), and infectious (32- 35) diseases. More recently it has been shown that the presence of genetically-determined higher levels of IL-10 secretion is an important component of the genetic background to systemic lupus erythematosus (36) and to the outcome of infectious disease (37). It has also been demonstrated that IL-10 secretion, resulted from *in vitro* stimulation of human peripheral blood leukocytes with LPS, varies markedly between individuals and that cytokine haplotypes are associated with different secretion levels (38). In addition, differences in IL-10 serum production by cells of patients and of their first-degree family members (37, 39), as well as differences in the distribution of IL-10 alleles, suggested the involvement of the different isoforms of the IL-10 gene as an important quantitative trait loci for human disease in infections (37, 40), autoimmune (26, 36, 41, 42) and malignant diseases (43).

The present inventors initially analyzed LPS-, Flu-, and amyloid peptide- specific IL-2 and IL-10 production by peripheral blood mononuclear cells (PBMC) of AD patients and age matched HC. Results showed that: 1) IL-2 production by PBMC of AD patients and controls was similar in all the conditions measured; and 2) IL-10 generation by LPS- and Flu -stimulated PBMC was comparably similar amongst the two groups of individuals. In contrast, an amyloid-specific immune impairment characterized by a reduced generation of IL-10 was present in AD. The observation that this cytokine imbalance was not seen in mitogen-stimulated PBMC indicates that amyloid-specific immune responses are selectively impaired in AD patients. Additionally, results showing that flu-stimulated proliferation was similar in patients and controls indicates that antigenic processing and presentation in association with HLA class II molecules, and the CD4-HLA class II self-restricted pathway of activation of the immune system (44), are not defective in these patients.

Next polymorphisms were analyzed in the IL-10 gene in the same group of subjects. Results stemming from analysis of the distribution of the IL-10 alleles in this Italian sample of healthy individuals showed a close similarity to those reported for other caucasian populations (45). In contrast, we observed a significantly higher frequency of the genotypes corresponding to reduced IL-10 production (ACC/ACC, ACC/ATA and ATA/ATA) in AD patients. Thus, an abnormally augmented prevalence of low-IL-10 producing isoforms in the AD population was determined; the phenotypic correlate of these isoforms becomes evident when amyloid-specific immune responses were measured.

Subsequent analyses focused on possible correlations between impaired IL-10 production and the clinical manifestations of AD by verifying whether the presence of low/intermediate IL-10 producing genotypes would be are associated with different disease outcomes. Results confirmed this to be the case. Thus, the presence of the ATA/ATA and of the GCC/ATA genotypes was correlated with an earlier age at disease onset. Additionally, the ACC/ATA and the ACC/ACC (all these are low/intermediate IL-10-producing genotypes) alleles were associated with a more severe cognitive impairment as indicated by a lower MMSE score.

It is interesting to observe that a recent report on Italian centenarians, individuals who - by definition - are less prone to develop age-related diseases, has demonstrated that extreme longevity is associated with a significantly higher frequency of the high IL-10-producing genotypes (46).

IL-10 is known to have potent anti-inflammatory properties (47); a biological scenario could thus be hypothesized in which the reduction of amyloid-specific IL-10 production would favour the triggering of the chronic inflammatory process seen in the progression of AD. These results suggest that an amyloid-specific and IL-10-mediated inhibitory feed-back circuit may be active in non-AD individuals; the rupture of this circuit could be associated with or predictive for the development of AD. Recently, a convincing study showed that an IL-10/pro-inflammatory circuit that revolves around cells of the innate immune system regulates susceptibility to autoimmune diseases (48). These results are expanded by showing that an alteration of this circuit is present in AD patients.

The present inventors have identified polymorphic regions, which polymorphs are indicative of a dysfunction of cytokine production and hence are associated with a predisposition towards an autoimmune, neurodegenerative or chronic inflammatory disease.

At present, Alzheimer's disease is diagnosed by recognised criteria such as DMS IV or NINCDS-ADRDA (23), often in conjunction with a magnetic resonance image (MRI) or computer aided tomography (CT) scan of the brain to identify the characteristic amyloid plaques and neurofibrillary tangles together with atrophy of the hippocampal area of the brain.

A definitive confirmatory diagnosis of Alzheimer's disease is only possible by a visual inspection of the affected areas of the brain during a post-mortem examination or via brain biopsy (not recommended due to lack of effective therapies).

Therapies and methods for monitoring of Alzheimer's disease are being urgently sought. As the progress is made in efforts to prevent or delay neurodegeneration and disease progression, early detection of Alzheimer's and identification of susceptible patients will gain importance as this will allow preventive measures being employed as early as possible. Therefore a need exists to be able to provide predictive and reliable tests for susceptibility to Alzheimer's disease without the need for lengthy and subjective assessments of cognitive performance.

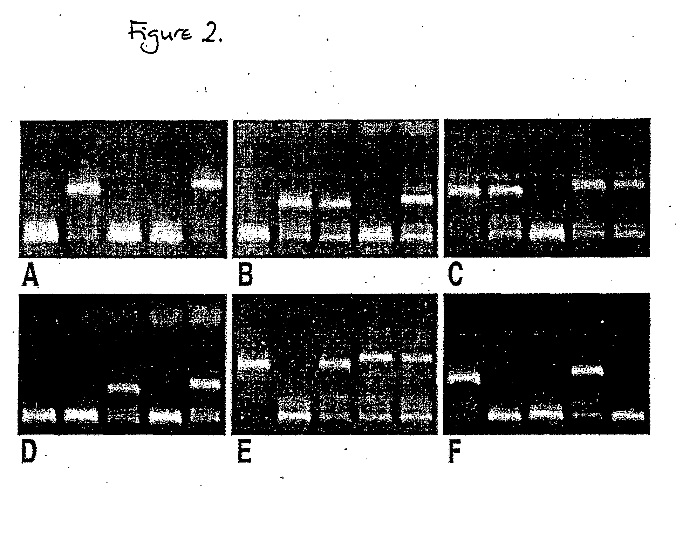

Accordingly, the present invention provides a method of determining the existence of or a predisposition to Alzheimer's disease, autoimmune disease or other neurodegenerative diseases, the method comprising the steps of taking a DNA bearing sample from a subject animal and analysing the sample to determine the allelic variants present at one or more of the SNP loci at positions -1082, -819 and -592 of the gene encoding IL-10, or to put it another way, analysing the sample for the presence or absence of the alleles of Figure 2.

Preferably, the genotype at all three positions -1082, -819 and -592 is determined.

While the identification of the alleles of Figure 2 has been found to be useful or predictive in the identification of Alzheimer's disease, a combination of the alleles of IL-10 and IL-6 has been found to be more strongly predictive of a predisposition to Alzheimer's or diagnostic of the presence of Alzheimer's disease.

Apolipoprotein E (Apo-E) has been associated with sporadic or non-familial AD. Hence, in a further aspect of the invention, a method of diagnosing Alzheimer's disease comprises the steps of obtaining a DNA-bearing sample from an animal and identifying the presence of a polymorphic allele of IL-10, IL-6 and of Apo-E.

Preferably, the polymorphic allele is one of the alleles of Figure 2.

Additionally, the sample may be assayed for the presence/absence of polymorphisms or other allelic variations of other cytokines in addition to IL-10 and IL-6, for example, IL-10 and IL-6 plus IL-4 and/or IL-1.

Alternatively, the sample may be assayed for the presence of absence of polymorphisms or other allelic variations of IL-10 plus Apo-E, or IL-6 plus Apo-E.

An interleukin 1 alpha (IL-1 alpha) polymorphism has been associated with Alzheimer's disease (77). Hence, in still further aspect of the invention, a method of diagnosing Alzheimer's disease comprises the steps of obtaining a DNA-bearing sample from an animal and identifying the presence of a polymorphic allele of IL-10, IL-6, Apo-E and of IL-1.

Generally, optimal predictive value will be obtained by combining as many predictive factors as possible in the test. The methods described herein together with markers such as Apo-E and IL-1 enable the development of a powerful diagnostic method that would include all the biological markers shown to have a predictive value toward the development of AD.

Accordingly, the present invention further provides a method of screening for compounds which modulate IL-10 implicated in Alzheimer's disease, the method comprising introducing the compound to be screened to DNA or cDNA fragments encoding the allelic polymorphisms of Table I, or to put it another way the allelic polymorphisms of Figure 2 and assessing the hybridisation between the compound and the fragment.

Hence, the present invention also provides compounds which modulate Alzheimer's disease, as identified by the above method.

Preferably, the animal is a mammal and more preferably a human being.

The data presented herein support the role of inflammatory processes in the pathogenesis of AD; reinforce the hypothesis that in AD patients neurodegeneration is tightly associated with an aberrant antigen-specific immune response; and lend further support to the use of anti-inflammatory compounds in the therapy of this disease.

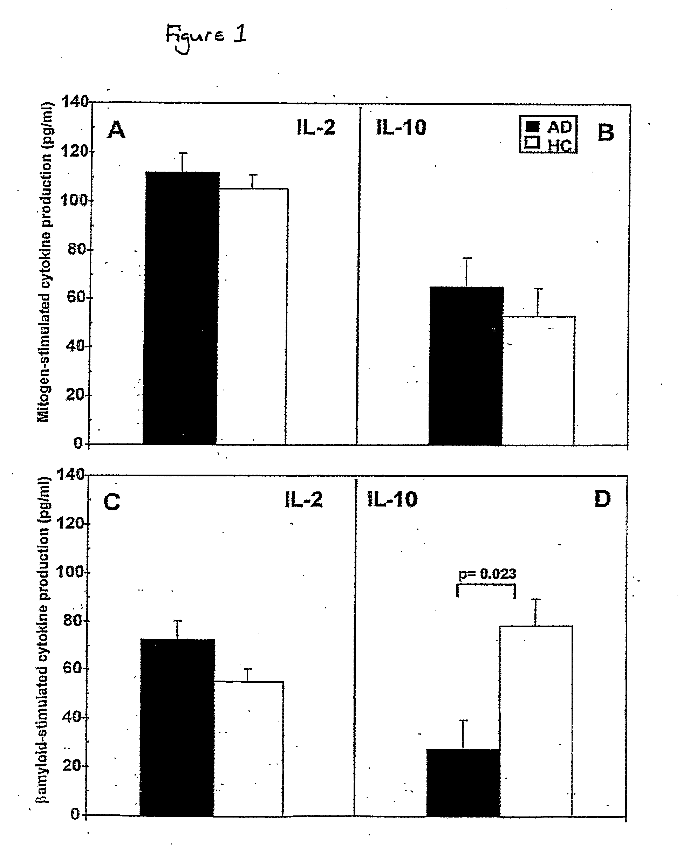

Embodiments of the invention will now be described by way of example only, with reference to the accompanying drawings of which
Figures 1A-1D are bar charts in which show LPS- and βamyloid- (a pool of 3β amyloid peptides: βA: fragments 25-35; βA: fragment 1-40; and βC: fragment 1-16) stimulated IL-2 (panels A and C) and IL-10 (panels B and D) production by PBMC of 47 AD patients (O) and 25 age- and sex-matched healthy controls (O). Mean values + standard errors are shown. p≤0.023;
Figure 2 shows paradigmatic example of IL-10 genotyping for six different samples. In each gel the heaviest bands correspond to the amplicons of the human β-globin gene which is used as the internal controls. The other specific amplified DNA fragments correspond to the polymorphisms of the IL-10 gene: GCC/GCC (A), GCC/ACC (B), GCC/ATA (C), ACC/ACC (D), ACC/ATA (E), ATA/ATA (F), and

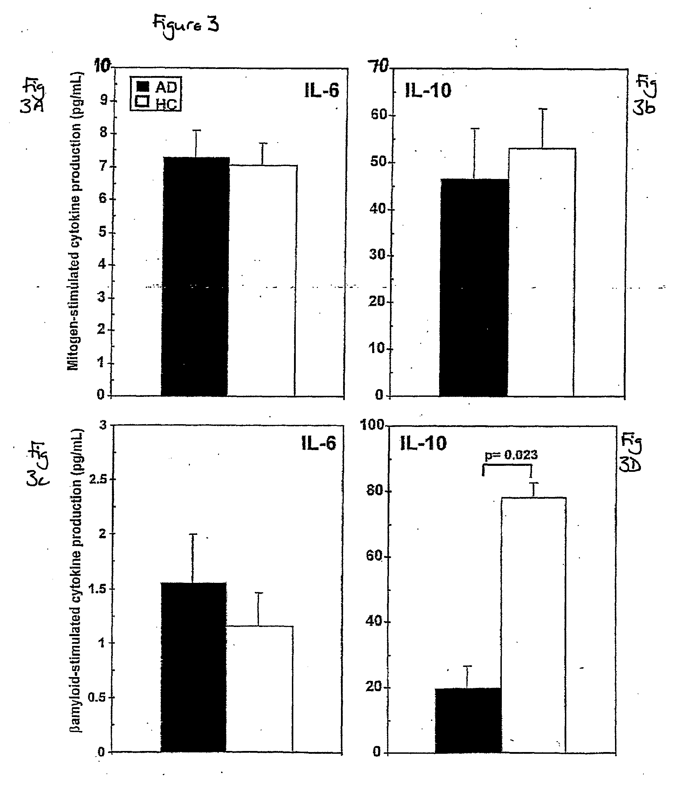

Figures 3A-3D are bar charts which show LPS- and β-amyloid-stimulated (a pool of three β-amyloid peptides; βA, fragment 25-35; βB, fragment 1-40; and βC, fragment 1-16) production of IL-6 (panels A and C) and IL-10 (panels B and D) by PBMC of 47 AD patients (O) and 25 age- and sex- matched healthy controls (O). Means + standard errors; p≤0.023.

### Example 1

### Patients and controls

Forty-seven AD patients and 25 non-demented subjects (HC) were included in a study of Alzheimer's disease. These patients were selected from a larger population sample followed at the Geriatric Department of the Ospedale Maggiore IRCCS, University of Milan, Italy. The DMS IV and NINCDS-ADRDA (23) criteria were adopted to obtain the clinical diagnosis of AD. Cognitive performances and alterations were assessed according to the Mini-Mental State Evaluation (MMSE). AD patients and HC were living at home and were carefully physical examined on the day of blood collection and their clinical records evaluated. In order to minimize the risk of clinical or sub-clinical inflammatory processes, all the patients were selected as follows: only AD and HC without clinical sign of inflammation (e.g. normal body temperature or absence of concomitant inflammatory disease) were included in the study. Blood chemical parameters were also evaluated and subjects with abnormal sedimentation rate of red blood cells or altered blood profile of albumin and transferring plasma levels were excluded. A further selection of AD patients were performed according to the C reactive protein (CRP) plasma levels and those patients with CRP above 5 mg/l (mean value ± 2 standard deviations of control values) were not enrolled in the study.

Informed consent to perform the study was obtained from controls and a relative of each AD patient.

### Blood sample collection

Whole blood was collected by venipuncture in Vacutainer tubes containing EDTA (Becton Dickinson Co, Rutherford, NJ). Peripheral blood mononuclear cells (PBMC) were separated by centrifugation on lymphocyte separation medium (Organon Teknika Corp., Durham, NC) and washed twice in PBS. The number of viable lymphocytes was determined by trypan blue exclusion and a hemocytometer.

### In vitro cytokine production

PBMCs were resuspended at 3x10⁶/ml in RPMI 1640 and were either unstimulated or stimulated with LPS (Sigma, St. Louis, MI)(10 g/ml), with a pool of 3 different peptides from the b-amyloid protein as follows: b-A: fragment 25-35 (25 mg/ml); b-B: fragment 1-40 (150 ng/ml); b-C: fragment 1-16 (150 ng/ml) (Sigma, St. Louis, MI); or with influenza virus vaccine (A/Taiwan+A/Shanghai+B/Victoria)(24 g/l; final dilution 1:1000)(Flu)(control antigen) at 37°C in a moist, 7% CO₂ atmosphere. Supernatants were harvested after 48 hours for LPS stimulation and after 5 days of culture for the b-amyloid protein peptides and Flu. Production of IL-2 and IL-10 by PBMCs was evaluated with commercial available ELISA kits (ACCUCYTE, Cytimmune Sciences, Inc, College Park, MD). All test kits were used following the procedures suggested by the manufacturer.

### IL-10 genotyping

Genomic DNA was extracted from EDTA-treated peripheral blood (10 ml) using a standard proteinase K and phenol/chloroform method. The DNA concentration and purity were determined by spectrophotometric analysis. A polymerase chain reaction-sequence specific primers (PCR-SSP) methodology was utilised to assess the IL-10 genotypes. The amplification of the sequence in the promoter region of the IL-10 (polymorphic positions - 1082, -819, -592) gene were performed using the Cytokine genotyping Tray Method (One Lambda, Canoga Park, CA, USA); the human β-globin gene was amplified as an internal control of genomic DNA preparation. PCR condition were indicated by One Lambda PCR program (OLI-1); the PCR products were then visualised by electrophoresis in 2.5% agarose gel.

### Statistical analysis

Statistical analysis was conducted using SPSS statistical package (SPSS, Chicago, IL). Differences in IL-10 production stemmed from analytic procedures based on non parametric analyses (Mann-Whitney); comparisons between different groups of patients were made using Fisher's exact 2-tailed test. Genotype frequencies were compared between the study groups by c² test with an observed significance level of the test below 0.05. Comparisons between the mean values of the age at onset and MMSE in the six different groups of AD were performed by one-way ANOVA analysis.

### Age, gender and MMSE scores in AD patients and in HC

Forty-seven AD patients and 25 age-matched healthy controls were enrolled in the study. The Mini-Mental State Evaluation (MMSE) showed the presence of a mild-to-severe cognitive deterioration in the AD patients. These data are shown in Table I.

### MBP-stimulated IL-10 production is reduced in AD patients

PBMC of 47 AD patients and of 25 age-and sex-matched HC were stimulated with a mitogen (LPS); a pool of 3 amyloid peptides ( A: fragment 25-35, B: fragment 1-40, and C: fragment 1-16)( amyloid), or Flu (used as a control antigen) and the production of IL-2 and IL-10 was measured with ELISA methods. No differences were seen when LPS- or Flu-stimulated IL-2 and IL-10 production was compared in AD patients and HC. amyloid-stimulated IL-2-production was also similar in the two groups of individuals studied. In contrast with these results, amyloid-stimulated production of IL-10 was significantly reduced (p= 0. 023) in AD patients compared to controls. These data are shown in Figure 1.

### The distribution of high, intermediate, and low IL-10 producing genotypes is skewed in AD patients

Paradigmatic example of the six different IL-10 genotypes, evaluated by PCR-SSP, is showed in Fig. 2 and their relative distribution among a typical Caucasian population sample is shown in Table II. In contrast with the distribution observed in HC, the frequency of the different IL-10 genotypes among AD patients was significantly skewed (c² = 16.007 with p=0.007) (Table II). Therefore genotypes corresponding to reduced IL-10 production (ACC/ACC, ACC/ATA and ATA/ATA genotypes) had a significantly higher distribution amongst AD subjects (17%, 26% and 11% respectively versus 4%, 16% and 4% in HC). Moreover the GCC/ACC to GCC/ATA ratio (intermediate phenotype) was 1:1 in AD while was 3:1 in HC.

### Low IL-10 production is correlated with worsened clinical outcome of AD

To analyse possible clinical correlates of the presence of low IL-10 genotype, we subsequently examined the six genotypes in relation to age of AD onset (Table III) and the progression of cognitive deterioration (Table IV). The results confirmed that the presence of low-IL-10-producing genotypes is indeed associated with a worsened clinical outcome of AD. Thus, presence of the ATA/ATA and GCC/ATA genotypes was associated with an earlier age at disease onset (ANOVA: p=0.042)(Table III); additionally, an inverse correlation was detected between ACC/ATA and ACC/ACC, low IL-10-producing genotypes, and the MMSE score (ANOVA: p=0.010)(Table IV).

**Table IV. Genetic Association Data for Autoimmune/Inflammatory Disease www.grc.nia.nih.gov/branches/rrb/dna/geneticdata.htm**

| **Chrom** | **CH-band** | **Gene** | **Disease** | **Allele** | **P-value** | **Reference** | **PubMedID** |
|---|---|---|---|---|---|---|---|
| 1 | 1q31.1 | CD45 | Ms | C to G in position 77 of PTPRC exon 4. | P=1.510-4 | Jacobsen M 00 | 11101853 |
| 1 | 1q31.1 | CD45 | SCld | deletion | na | Kung C 00 | 10700239 |
| | Mouse | CD45 | autoimmune nephritis | glutamate 613 to arginine | na | Majeti R 00 | 11163182 |
| | | | | | | | |
| 1 | 1q32.1 | IL10 | SLE | -4kb to 5' | P=.0001 | Gibson AW 01 | 11238636 |
| 1 | 1q32.1 | IL10 | SS | -10 GCC haplotype (G -1082, C -819, and C -592 of the IL-10 gene | P=<0.05 | Hulkkonen J 01 | 11212157 |
| 1 | 1q32.1 | IL10 | RA | genotype-1082GG | P=<0.03 | Huizinga TW 00 | 11085795 |
| 1 | 1q32.1 | IL10 | RA | ATA haplotype, pts w/>4 joints | P=0.02 | Crawley E 99 | 10366102 |
| 1 | 1q32.1 | IL10 | GVHD | IL-10 (-)1064 | P=<.001 | Middleton PG 98 | 9808588 |
| 1 | 1q32.1 | IL10 | IBD/UC | -1082*G allele (high producer) was reduced in pts | P=0.03 | Tagore A 99 | 10551422 |
| | | | | | | | |
| 2 | 2q12.2 | IL1RA | SLE | IL1RN*2 allele | na | Blakemore AL 94 | 7945503 |
| 2 | 2q12.2 | IL1RA | Ulcerative Colitis | IL1RN*2 allele | P=0.007 | Mansfield JC 94 | 8119534 |
| 2 | 2q12.2 | IL1RA | polymyalagia rheumatica | IL1RN*2 allele | na | Boiardi L 00 | 11138328 |
| | | | | | | | |
| 2 | 2q33.1 | CTLA4 | RA | A/G 49 | P=0.009 | Gonzalez MF 99 | 10203024 |
| 2 | 2q33.1 | CTLA4 | GD | A/G 49 | P=<0.01 | Yanagawa T 97 | 9459626 |
| 2 | 2q33.1 | CTLA4 | MS | A/G 49 | P=0.006 | Harbo HF 99 | 10082437 |
| 2 | 2q33.1 | CTLA4 | H-Thy | A/G 49 | P=<0.03 | Donner H 97 | 9398726 |
| 2 | 2q33.1 | CTLA4 | IDDM | A/G 49 | P=0.004 | Takahiro A 99 | |
| 2 | 2q33.1 | CTLA4 | IDDM | | na | Yanagawa T 99 | 10052685 |
| 2 | 2q33.1 | CTLA4 | IDDM | A/G 49 | P=0.00002 | Marron MP 97 | 9259273 |
| | | | | | | | |
| 5 | 5q31.1 | IL4 | GD | position 590 allele reduced in GD | P=0.00004 | Hunt PJ 00 | 10843185 |
| 5 | 5q31.1 | IL4 | increased IgE | C+33T polymorphism with elevated total serum IgE | P=<0.05 | Suzuki I 00 | 11122213 |
| 5 | 5q31.1 | IL4 | asthma, FEV(1) | C-589T IL-4 promoter genotype (TT) | P=0.013 | Burchard EG 99 | 10471619 |
| 5 | 5q31.1 | IL4 | AD | -590C/T | P=0.001 | Kawashima T 98 | 9643293 |
| 5 | 5q31.1 | IL4 | RA | IL-4(2) higher in non-destructive RA | P=0.0006 | Buchs N 00 | 11035134 |
| 5 | 5q31.1 | IL4 | MS | IL-4 B1 allele, late onset MS | P=<0.001 | Vandenbroeck K 97 | 9184650 |
| | | | | | | | |
| 5 | 5q31.1 | IL13 | asthma | Gln110Arg | P=0.017 | Heinzmann A 00 | 10699178 |
| 5 | 5q31.1 | IL13 | asthma | C to T at position-1055 (TT) | P=0.002 | van der Pouw Kraan TC 99 | 11197307 |
| | | | | | | | |
| 6 | 6p21.31 | TNFa | asthma | G/A-308TNF2 | P=0.003 | Albuquerque R 98 | 9645594 |
| 6 | 6p21.31 | TNFa | PrimBilCirr | G/A-308 TNF1 | P=0.02 | Gordon M 99 | 10453936 |
| 6 | 6p21.31 | TNFa | Sepsis | G/A-308TNF2 | P=0.007 | Majetschak M 99 | 10450735 |
| 6 | 6p21.31 | TNFa | Psoriasis | G/A-308 TNF1 | P=2.74 X 10-8 | Arias A 97 | 9395887 |
| 6 | 6p21.31 | TNFa | lep. Leprosy | G/A-308 | P=.02 | Roy S 97 | 9237725 |
| 6 | 6p21.31 | TNFa | GVHD | TNFd | P=.006 | Middleton PG 98 | 9808588 |
| 6 | 6p21.31 | TNFa | Silicosis | G/A -308 TNF1 | P=<0.05 | Yucesoy B 01 | 11264025 |
| 6 | 6p21.31 | TNFa | SLE | G/A -308 TNF1 | na | Sullivan KE 97 | 9416858 |
| 6 | 6p21.31 | TNFa | celiac | G/A -308 TNF1 | P=<0.001 | McManus R 96 | 8655356 |
| 6 | 6p21.31 | TNFa | chronic bronchitis | G/A -308 TNF1 | P=<0.01 | Huang S 97 | 9372657 |
| 6 | 6p21.31 | TNFa | Psoriasis | -238 TNF1 | P=1.64X 10-7 | Arias A 97 | 9395887 |
| | | | | | | | |
| 7 | 7p15.3 | IL6 | IDDM | G,G(-174) increased in pts | P=<0.002 | Jahromi MM 00 | 11054276 |
| 7 | 7p15.3 | IL6 | SLE | AT-rich minisatellite in 3' flanking region | P=0.001 | Linker-Israeli M 99 | 11197305 |
| 7 | 7p15.3 | IL6 | RA | 622 and-174 alleles, age of onset | na | Pascual M 00 | 11196696 |
| 7 | 7p15.3 | IL6 | MS | carriage larger alleles A6-->A9, accelerated onset | P=0.025 | | |
| | | | | | | | |
| 12 | 12q12 | VDR | GD | exon 2 initiation codon (VDR-FOK:I) polymorphism | P=0.023 | Ban Y 00 | 11134121 |
| 12 | 12q12 | VDR | RA | BB/tt genotype | na | Garcia-Lozano JR 01 | 11251690 |
| 12 | 12q12 | VDR | MS | bb | P=0.0263 | Fukazawa T 00 | 10465499 |
| 12 | 12q12 | VDR | CD | tt | P=0.017 | Simmons JD 00 | 10896912 |
| 12 | 12q12 | VDR | IDDM | Bsml | P=0.015 | Chang TJ 00 | 10792336 |
| | | | | | | | |
| 12 | 12q21.1 | IFNG | asthma | CA repeat polymorphism within the first intron | P=.0018 | Nakao F 01 | 11240951 |
| 12 | 12q21.1 | IFNG | IDDM | CA repeat polymorphism within the first intron | P=0.039 | Awata T 94 | 7867888 |
| 12 | 12q21.1 | IFNG | GD | CA repeat polymorphism within the first intron | P=<0.04 | Siegmund T 98 | 9848715 |
| 12 | 12q21.1 | IFNG | RA | CA repeat polymorphism within the first intron | P=<0.0001 | Khani-Hanjani A 00 | 11022930 |
| | | | | | | | |
| 16 | 16p11.1 | IL4R | asthma | Ile50Val | P=<0.0001 | Mitsuyasu H 98 | 9620765 |
| 16 | 16p11.1 | IL4R | hyper-IgE syndrome and severe eczema, atopy | Arg576G | P=0.001 | Hershey GKK 97 | 9392697 |
| 16 | 16p11.1 | IL4R | MS(PPMS) | IL4R variant R551 | P=0.001 | Hackstein H 01 | 11164908 |
| | | | | | | | |

| | | | | | | | |
|---|---|---|---|---|---|---|---|
| [DNA Array Unit] [IRP Home] [NIA Home] | | | | | | | |

### Example 2

### Patients and controls

Sixty-five AD patients (44 F/21 M, mean age 80±2) and 65 non-demented sex- and age- matched healthy controls (HC) were enrolled. The patients were selected from a larger population sample followed at the Geriatric Department of the Ospedale Maggiore IRCCS, University of Milan, Italy. We applied the DMS IV and NINCDS-ADRDA (23) criteria to obtain the clinical diagnosis of AD; every subject had a recent brain magnetic resonance imaging (MRI)/computed tomography (CT) scan available. Cognitive performances and alterations were assessed according to the Mini-Mental State Evaluation (MMSE). AD patients and HC were living at home and a careful physical examination was done on the day of blood collection, and their clinical records were consulted.

In order to minimize the risk of clinical or sub-clinical inflammatory processes, subjects were selected as follows: only AD and HC without clinical signs of inflammation (e.g. normal body temperature, no concomitant inflammatory condition) were eligible. Blood chemistry tests were done and subjects with an abnormal red blood cell sedimentation rate or altered albumin and transferring plasma levels were excluded. AD patients were further selected according to their C reactive protein (CRP) plasma levels and any with CRP above 5 mg/L (mean + 2 standard deviations of control values) were not eligible.

Informed consent was obtained from all the subjects or their relatives. The study protocol was approved by the Ethics Committee of the University Hospital.

### Blood sampling

Whole blood was collected by venipuncture in Vacutainer tubes containing EDTA (Becton Dickinson Co., Rutherford, NJ). Peripheral blood mononuclear cells (PBMC) were separated by centrifugation on lymphocyte separation medium (Organon Teknika Corp., Durham, NC) and washed twice in PBS. Viable lymphocytes were counted by Trypan blue exclusion and a hemocytometer.

### Genotyping

Genomic DNA was extracted using a standard proteinase K and phenol/chloroform method. The DNA concentration and purity were determined by spectrophotometric analysis. A polymerase chain reaction-sequence-specific primers (PCR-SSP) method was utilised to assess IL-10 and IL-6 genotypes. The sequence in the promoter region of the IL-10 (polymorphic positions -1082, -819, -592) and IL-6 (polymorphic position -174) genes was amplified using the cytokine genotyping tray method (One Lambda, Canoga Park, CA, USA); the human β-globin gene was amplified as an internal control for the genomic DNA preparation. PCR conditions were indicated by the One Lambda PCR program (OLI-1) and the PCR products were visualised by electrophoresis in 2.5% agarose gel.

The ApoE genotypes were determined by PCR amplification of a 234 base-pair fragment of exon 4 of the ApoE gene, followed by digestion with Cfo1. The restriction patterns were obtained by gel electrophoresis.

### In vitro cytokine production

PBMCs were resuspended at 3x10⁶/mL in RPMI 1640 and were either unstimulated or stimulated with LPS (Sigma, St. Louis, MI)(10µg/mL), with a pool of three peptides from the β-amyloid protein as follows: β-A, fragment 25-35 (25 g/mL); β-B, fragment 1-40 (150 ng/mL); β-C, fragment 1-16 (150 ng/mL)(Sigma), or with influenza virus vaccine (A/Taiwan+A/Shanghai+B/Victoria) (24 µg/L; final dilution 1:1000) (Flu) (control antigen) at 37°C in a moist, 7% CO₂ atmosphere. Supernatants were harvested after 48 hours for LPS stimulation and after five days of culture for the β-amyloid protein peptides. Production of IL-10 and IL-6 by PBMCs was evaluated with commercial ELISA kits (ACCUCYTE, Cytimmune Sciences Inc., College Park, MD). All test kits were used following the manufacturer's directions.

### Statistical analysis

Statistical analysis was done using the SPSS statistical package (SPSS, Chicago, IL). Genotype frequencies were compared in the study groups by the χ² test with a level of significance below 0.05. The odds ratio (OR) and 95% confidence intervals (CI were also calculated. Adjusted ORs were estimated by logistic regression, controlling for ApoE 4 carrier status. Homogeneity of the ORs between strata was assessed by including the appropriate interaction terms in the model. Differences in IL-10 and IL-6 production were established by procedures based on non-parametric analysis (Mann-Whitney); different groups of patients were compared using Fisher's exact two-tailed test.

### The distribution of high, intermediate, and low IL-10 producing genotypes is skewed in AD patients

The genotype and allele frequencies of the biallelic polymorphism at position -1082 are reported in Table V. This SNP alters transcriptional activation with a gene dosage-related effect, so GG genotype correlates with high, GA with intermediate and AA with low IL-10 production after stimulation of T cells *in vitro* (57). AD patients show a significantly higher frequency of the -1082A low producer allele, which skews the genotype distribution in AD compared to HC with a significant decrease of -1082GG high producer genotype (Table V).

**Table V. Frequency of the different IL-10 genotypes and alleles observed in Alzheimer's disease patients (AD) and in healthy age-matched controls.**

| Genotype | | | | Allele | |
|---|---|---|---|---|---|
| | G/G (H)^{a} | G/A (M) | A/A (L) | A | G |
| **AD** | 4 (6.4%) | 28 (44.4%) | 31 (49.2%) | 90 (71.4%) | 36 (28.6%) |
| **HC** | 14 (22.2%) | 29 (46%) | 20 (31.8%) | 69 (54.8%) | 57 (45.2%) |

| | | | | | |
|---|---|---|---|---|---|
| *^{a}* The corresponding phenotypes: high (H), intermediate (M), low (L) are shown in brackets Genotype: χ² = 7.946 , df= 2 , p= 0.019 Allele: χ² = 6.817 , df= 1 , p= 0.009 | | | | | |

The some SNP is linked with two other SNPs at positions -819 and -592. They combine with microsatellite alleles to form haplotypes where the difference in IL-10 production is mainly accounted for by the -1082 SNP (38, 42). The genotype and allele frequencies of -819 C→T and -592 C→A SNPs were distributed similarly in our AD and HC samples (data not shown).

### The -174C allele in the IL-6 gene is over-represented in AD patients

The distribution of IL-6 genotypes and alleles in HC and AD is shown in Table 6. This functional polymorphism also seemed related to the plasma IL-6 concentration; however, it is not clear how this SNP influences IL-6 plasma levels (54). The results of the genotype distribution in our AD and HC samples, with a lower frequency of GG genotype in AD patients. Similarly, the allele distribution was significantly different in the two groups, the C allele being significantly higher in AD (Table VI).

**Table VI. Frequency of the different IL-6 genotypes and alleles observed in Alzheimer's disease patients (AD) and in healthy age-matched controls.**

| Genotype | | | | Allele | |
|---|---|---|---|---|---|
| | G/G (H)^{a} | G/C (H) | C/C (L) | C | G |
| **AD** | 17 (29%) | 34 (57.6%) | 8 (13.4%) | 50 (42.4%) | 68 (57.6%) |
| **HC** | 32 (50%) | 27 (42.2%) | 5 (7.8%) | 37 (28.9%) | 91 (71.1%) |

| | | | | | |
|---|---|---|---|---|---|
| *^{a}* High (H) and low (L) phenotypes are in brackets Genotype: χ² = 5.894 , df= 2 , p= 0.052 Allele: χ² = 4.300 , df= 1, p= 0.038 | | | | | |

### L-10 and IL-6 allele combination and relative risk of developing AD

We investigated whether any combination of the IL-10 GA and IL-6 GC alleles affected the risk of AD. The concomitant presence of both IL-10 A and IL-6 C alleles significantly raised this risk, independently of the ApoE4 status (Table VII). The IL-10 A/A genotype alone or the IL-6 C/C genotype alone both conferred a smaller increase in the risk of the disease (OR 5.8, Cl 1.7-20, p=0.005; OR 3.0, CI 0.9-10.6, p=0.087).

**Table VII. IL-10 and IL-6 alleles and risk for Alzheimer disease**

| IL-10 | IL-6 | OR | 95% CI | adj. OR | 95% Cl |
|---|---|---|---|---|---|
| **G allele** | **G allele** | 1 | | 1 | |
| **G** | **C** | 2.8 | 0.2-40 | 0.9 | 0.1-26.5 |
| **A** | **G** | 4.6 | 0.5-41 | 3.3 | 0.3-36.3 |
| **A** | **C** | 11.2* | 1.3-97.3 | 10.3* | 1.0-108 |

| | | | | | |
|---|---|---|---|---|---|
| 7*p>0.05; OR: crude odds ratio; adj. OR: apolipoprotein E ε4 adjusted odds ratio; CI: confidence interval | | | | | |

### LPS, Flu, and amyloid peptide-stimulated IL-10 and IL-6 production is reduced in AD patients

PBMC of 47 AD patients and 25 age- and sex-matched HC were stimulated with a mitogen (LPS), with a pool of three β amyloid peptides (βA, fragment 25-35; βB, fragment 1-40; βC, fragment 1-16), or with Flu and the production of IL-10, IL-6 was measured with ELISA methods. There were no differences in LPS- or flu- stimulated IL-6 and IL-10 production in AD and HC. In contrast, when β-amyloid-stimulated production of IL-6 and IL-10 was analysed, a marginal increased IL-6 production and a significant decrement of IL-10 generation (p= 0.023) were seen in AD patients compared to HC, suggesting an antigen-specific impairment in the production of these cytokines. These data are shown in Figure 3.

The causative role of chronic inflammation in the pathogenesis of AD is still mainly speculative (24, 25). Nonetheless a "cytokine cycle" has been proposed where (19) the anti-inflammatory cytokines (IL-4, IL-10 and IL-13) regulate β-amyloid-induced microglial/macrophage inflammatory responses and modify the microglial activity surrounding amyloid neuritic plaques (52). These cytokines can inhibit the induction of IL-1, TNF-α and MCP-1 in differentiated human monocytes and, above all, IL-10 causes dose-dependent inhibition of the IL-6 secretion induced by β-amyloid in these cells and in murine microglia (19).

From a clinical point of view, IL-10 is involved in autoimmune diseases (41, 42, 26) and in malignancies (31, 27, 43) where the higher levels of the cytokine depend on genetic background (59) but also influence the outcome of infections (34, 40, 37).

More consistent is the evidence of a role of IL-6 in the pathogenesis of AD. Elevated IL-6 immunoreactivity was observed close to amyloid plaques in the brain of these patients (67); IL-6 induces the synthesis of β-amyloid precursor protein (69), and in transgenic mouse models elevated CNS levels of IL-6 result in neuropathogenic effects and cognitive deficits (51).

The C allele of a VNTR on the IL-6 gene was reported to reduce cytokine activity (61). The IL-6 VNTR C allele has been correlated with a delayed initial onset and reduced AD risk in a German population (63). The functional polymorphism -178 of the promoter region could also be involved in the development of AD phenotype because of its association with plasma concentrations of the cytokine (54). However, in two clinical sets of different ethnic origin the results were debatable (49).

In our sample the data from SNP analysis showed HC had a distribution of IL-10 and IL-6 alleles similar to that of an Italian population (65). More importantly, the present results point to a significantly higher percentage of IL-10 -1082A carriers among AD cases. A recent report on Italian centenarians, who are clearly less prone than younger persons to age-related diseases, showed that extreme longevity is significantly associated with the high IL-10-producing genotypes (58).

As we have previously reported, the results on IL-6 SNPs are more contradictory. The IL-6 G allele seems significantly in AD of Japanese (66) and also of southern Italian origin (64), whereas in our sample it is the C allele that appears over-represented.

To link these differing findings several points have to be considered. Ethnicity may strongly influence the role of genetic risk factors, and so may the distribution of gene variants in the populations of different European countries, or even among different areas of the same country (53, 55, 60, 62, 70). In addition, the association between AD and IL-6 SNPs may be confined to particular ages, and in our samples AD and HC subjects were all old-old.

Finally, we must considered the role played by a gene or by several genes in linkage disequilibrium with this mutation: a strong disequilibrium between -174 SNP and the VNTR polymorphism of the 3' flanking region of the IL-6 gene has been described in Germans (49).

The main finding of this study was the identification of a group of subjects with a high risk of late-onset AD on account of the concomitant presence of IL-10 -1082A and IL-6 -174C alleles. We also explored interactions between Apo E and IL-10 or IL-6 genes but did not find any evidence of synergistic effects, suggesting that these inflammation- related alleles are an additional and independent risk factor for AD.

To shed more light on the genetic results, the inventors also analysed β-amyloid peptide-, LPS-, and Flu-specific IL-10 and IL-6 production by peripheral blood mononuclear cells (PBMC) in a subset of AD patients and age-matched HC. The results showed that: 1) IL-6 production by PBMC of AD patients and controls did not differ significantly in any conditions; and 2) IL-10 generation by LPS- and Flu-stimulated PBMC was comparable in the two groups, whereas a β-amyloid-specific immune impairment characterized by a reduced generation of IL-10 was noted in AD. The fact that this cytokine imbalance was not seen in mitogen-stimulated PBMC indicates that β-amyloid-specific immune responses are selectively impaired in AD. Additionally, the finding that flu-stimulated proliferation was similar in patients and controls indicates that antigenic processing and presentation in association with HLA class II molecules, and the CD4-HLA class II self-restricted pathway of activation of the immune system (44), are not defective in AD. Thus a biological scenario is conceivable in which the reduction of amyloid-specific IL-10 production favours the triggering of the chronic inflammatory process seen in the AD brain. An amyloid-specific and IL-10-mediated inhibitory feedback circuit could be active in non-AD individuals, and a breakdown of this circuit could be associated with, or predictive of, the development of AD. A recent study showed convincingly that an IL-10/proinflammatory circuit revolving around cells of the innate immune system regulates susceptibility to autoimmune diseases (48). Our results extend this concept by showing that in AD patients this circuit is altered. The data as a whole support the theory that the overall risk of developing AD may be governed by a "susceptibility profile", that reflects the combined influence of inheriting multiple high-risk alleles, and casts light on the pivotal role of IL-10 and IL-6 SNPs in this profile.

Inflammation is involved in the pathogenesis of Alzheimer's disease (AD, the anti-inflammatory cytokine interleukin-10 (IL-10) might counteract IL-6 activity in the brain. As the promoter of these genes is polymorphic, the 65 AD patients and 65 healthy controls (HC) the present investigated the IL-10 -1082 GA and IL-6 -174 GC alleles. In several cases they also assessed IL-10 and IL-6 production by PBMC. For IL-10 there was a significant higher level of the -1082GG genotype (p=0.019) in HD than HC, while for IL-6 the G/G genotype was lower and the C allele higher (p<0.005). The concomitance of IL-10 A and IL-6 C alleles significantly raised the risk of AD (odds ratio: OR 11.2, confidence interval: CI 1.3-97.3; p<0.05) independently of ApoE4 (adjusted OR 10.3, CI 1-108; p<0.05). Only amyloid-stimulated IL-10 production differed in AD and HC (p=0.023). These results conflict with the inflammatory theory in AD, pointing to a pivotal role of IL-10 and lL-6 polymorphisms and a selective alternation in this network.

### Example 3

### Genotype analyses on interferon-γ and TNF-α

The methods described in the preceding Examples were used to perform genotype analysis on interferon-γ and TNF-α in Alzheimer's patients and healthy controls. A summary of the results is shown in tables VIII and IX.

**Table VIII. IFN-γ genotype distribution**

| | Genotype (c) | | | Allele | |
|---|---|---|---|---|---|
| | T/T(H) | T/A(I) | A/A (L) | T | A |
| **AD** | 11(15.5%) | 35(49.3%) | 25(35.2%) | 57(40%) | 85(60%) |
| **HC** | 11(18%) | 31(51%) | 19(31%) | 53(43%) | 69(57%) |
| | | | | | |

| | | | | | |
|---|---|---|---|---|---|
| The frequencies of the different genotypes among Alzheimer's disease patients (AD) were not statistically different from those of the health controls (HC). χ² = 0.305, df= 2, p=0.859. In the brackets (c) there are the corresponding phenotype high (H), intermediate (M) and low (L). Allele: χ² = 0.174, df= 1, p=0.676_{.} | | | | | |

**Table IX. TNF-α genotype distribution**

| | **Genotype** | | | **Allele** | |
|---|---|---|---|---|---|
| | G/G (L)^{a} | G/A (H) | A/A (H) | C | G |
| **AD** | 60 (82%) | 12 (16.5%) | 1 (1.5%) | 132 (90%) | 14 (10%) |
| **HC** | 32 (69%) | 13 (28%) | 1 (3%) | 77 (84%) | 15 (16%) |

| | | | | | |
|---|---|---|---|---|---|
| The frequencies of the different genotypes among Alzheimer's disease patients (AD) were not statistically different from those of the health controls (HC) *^{a}* High (H) and low (L) phenotypes are in brackets Genotype: χ² = 2.568 , df= 2 , p= 0.277 Allele: χ² = 1.792, df= 1 , p= 0.181 | | | | | |

There are no statistically significant differences when Alzheimer's patients and controls are compared indicating that neither interferon-γ nor TNF-α is associated with the likelihood of developing Alzheimer's disease.

In contrast, the present invention shows that IL-10 and IL-6 are highly predictive for developing Alzheimer and possibly also predict disease progression. The best predictive value will be achieved by combining genotype tests for multiple gene polymorphisms *e.g*. IL10, IL-6, Apo-E and others shown to be associated with Alzheimer's disease.

### References

1. Ernst, R.L. and J.W. Hay. 1994. The US economic and social costs of Alzheimer's disease revised. Am. J. Public Health. 84: 1261.
2. Goate, A., M.C. Chartier-Harlin, M. Mullan, J. Brown, F. Crawford, L. Fidani, L. Giuffra, A. Haynes, N. Irving, L. James, R. Mant, P. Newton, K. Rook, P. Roques, C. Talbot, M. Pericak-Vance, A. Roses, R. Williamson, M. Rossor, M. Owen and J. Hardy.1991. Segregation of a missense mutation in the amyloid precursor protein gene with familial Alzheimer's disease. Nature. 349: 704.
3. Levy-Lahad, E., W. Wasco, P. Poorkaj, D.M. Romano, J. Oshima, W.H. Pettingell, C.E. Yu, P.D. Jondro, S.D. Schmidt, K. Wang, A.C. Crowley, Y.H. Fu, S.Y. Guenette, D. Galas, E. Nemens, E.M. Wiisman, T.D. Bird, G.D. Schellemberg and R.E. Tanzi. 1995. Candidate gene for the chromosome 1 familial Alzheimer's disease locus. Science. 269: 973.
4. Sherrington, R., E.I. Rogaev, Y. Liang, E.A. Rogaeva, G. Levesque, M. Uveda, H. Chi, C. Lin, G. Li, K. Holman, T. Tsuda, L. Mar, J.F. Fonci, A.C. Bruni, M.P. Montesi, S. sorbi, I. Rainero, L. Pinessi, L. Nee, I. Chumaken, D. Pollen, A. Brookes, P. Sanseau, R.J. Polinsky, W. Wasco, H.A.R. Da Silva, J.L. Haines, M.A. Pericak-Vance R.E. Tanzi, A.D. Roses, P.E. Fraser, J.M. Rommens and P.H. George-Hyslop. 1995. Cloning of a gene bearing missense mutation in early-onset familial Alzheimer's disease. Nature. 375: 754.
5. Blacker, D., J.L. Haines, L. Rodes, H. Terwedow, R.C.P. Go, L.E. Harrel, R.T. Perry, S.S. Basset, G. Chase, D. Meyers, M.S. Albert and R. Tanzi. 1997. ApoE-4 and age at onset of Alzheimer's disease: The NINH Genetics Initiative. Neurology. 48: 139.
6. Poirier, J., J. Davignon, D. Bouthillier, S. Kogan, p. Bertrand and S. Gauthier. 1993. Apolipoprotein E polymorphism and Alzheimer's disease. Lancet. 342: 697.
7. Saunders, A.M., W.J. Strittmatter, D. Schmechel, P.H. George-Hyslop, M.A. Pericak-Vance, S.H. Joo, B.L. Rosi, J.F. Gusella, D.R. Crapper-MacLachlan and M.J. Alberts.1993. Association of apolipoprotein E allele epsilon 4 with late-onset familial and sporadic Alzheimer's disease. Neurology.. 43:1467.
8. Fassbender, K., C. Masters and K. Beyreuther.2000. Alzheimer's disease: an inflammatory disease?. Neurobiology of Aging. 21: 433.
9. McGeer, P.L. and E.G. McGeer. 2001. Inflammation, autotoxicity and Alzheimer disease. Neurobiology of Aging. 22: 799.
10. Zandi, P.P and J.C.S. Breitner. 2001. Do NSAIDs prevent Alzheimer's disease? And, if so, why? The epidemiological evidence. Neurobiology of Aging. 22: 811.
11. Rogers, J., L.C: Kirby, S.R. Hempelman, D.L. Berry, P.L. McGeer, A.W. KasniaK, j. Zalinski, M. Cofield, L. Mansukhani, and P. Willson.1993. Clinical trial of indomethacin in Alzheimer's disease. Neurology. 43: 1609.
12. Hauss-Wegrzyniak, B., L.B. Willard, P. Del Soldato, G. Pepeu and G.L. Wenk. 1999. Peripheral administration of novel anti-inflammatories can attenuate the effects of chronic inflammation within the CNS. Brain Res.815:36.
13. Neuroinflammation Working Group. 2000. Inflammation and Alzheimer's disease. Neurobiology of Aging. 21: 383.
14. Licastro, F., S. Pedrini. L. Caputo, G. Annoni, L.J. Davis, C. Ferri, V. Casadei and L.M.E. Grimaldi. 2000. Increased plasma levels of interleukin-1, interleukin-6 and a-1-antichymotrypsin in patients with Alzheimer's disease: peripheral inflammation or signals from brain?. Journal of Neuroimmunology. 103: 97.
15. Mrak, R.E. and W.S.T. Griffin. 2001. Interleukin-1, neuroinflammtion, and Alzheimer's disease. Neurobiology of Aging. 22: 903.
16. Perry, R.T., J.S. Collins, H. Wiener, R. Acton and R.C.P. Go. 2001. The role of TNF and its receptors in Alzheimer's disease. ..Neurobiology of Aging. 22: 873.
17. Wyss-Coray, T., E. Masliah, M. Mallory, L. McConlogue, K. Johnson-Wood, C. Lin and L. Mucke. 1997. Amyloidogenic role of cytokine TGF-beta1 in trangenic mice and in Alzheimer's disease. Nature. 389: 603.
18. Meda, L., P. Baron, E. Prat, E. Scarpini, R. Delgado, A. Catania, J.M. Lipton and G. Scarlato. 1999. Proinflammatory profile of cytokine production by human monocytes and murine microglia stimulated with beta-amyloid. J. Immunol. 93: 45.
19. Szczepanik, A.M., S. Funes, W. Petko and G.E. Ringheim. 2001. II-4, IL-10 and II-13 modulate A beta (1--42)-induced cytokine and chemokine production in primary murine microglia and a human monocyte cell line. J. Neuroimmunol.. 113: 49.
20. Lombardi, V.R., M. Garcia, L. Rey and R. Cacabelos. 1999. Characterization of cytokine production, screening of lymphocyte subset patterns and in vitro apoptosis in healthy and Alzheimer's Disease (AD). J Immunol. 97: 163.
21. Engelborghs, S., M. De Brabander, J. De Cree, R. D'Hooge, H. Geerts, H. Verhaegen and P.P. De Deyn. 1999. Unchanged levels of interleukins, neopterin, interferon-gamma and tumour necrosis factor-alpha in cerebrospinal fluid of patients with dementia of the Alzheimer type. 34: 523.
22. Hutchinson, I.V., V. Pravica and P.J. Sinnot. 1998. Genetic regulation of cytokine synthesis: consequences or acute and chronic organ allograft rejection. Graft. 1: 15.
23. McKhann, G., D. Drachman, M. Folstein, R. Katzman, D. Proce, E.M. Stadlan. 1984. Clinical diagnosis of Alzheimer's disease. Neurology. 34: 939.
24. Eikelenboom, P., S.S. Zhan, W.A. van Goll and D. Allsop D. 1994. Inflammatory mechanisms in Alzheimer disease. Trends Pharmacol Sci 15:447.
25. Rogers, J., S. Webster, and L.F. Lue. 1996. Inflammation and Alzheimer's disease pathogenesis. Neurobiol Aging 17:686.
26. Llorente, L., W. Zou, Y. Levy, Y. Richaud-Patin, Y. Wijdenes, J. Alcocer-Varela, B. Morel-Fourrier, J.C. Brouet, D. Alarcon-Segovia, P. Galanaud. 1995. Role of interleukin 10 in the B lymphocyte hyperactivity and autoantibody production of human systemic lupus erythemattosus. J. Exp. Med. 181: 839.
27. Luscher, U., L. Filgueira, A. Juretic, M.Zuber, L.J. Luuscher, M. Heberer and g.C. Spagnoli. 1994. The pattern of cytokine gene expression in freshly excised human metastatic melanoma suggests a state of reversible anergy of tumor-infiltrating lymphocytes. Int. J. Cancer. 57: 612.
28. Matsuda, M., F. Slazar, M. Petersson, G. Masucci, J. Hansson, Q.C. Zhang, M.G. Masucci and R. Kiessling. 1994. Interleukin 10 pretreatment protects target cells from tumor- and allo-specific cytottoxic T cells and downregulates HLA class I expression. J. Exp. Med .180: 2371.
29. Kim, J., R.L. Modlin, R.L. Moy, S.M. Dubinett, T. McHugh, B.J. Nickloff and K. Uyemura. 1995. IL-10 production in cutaneous basal and squamos cell carcinomas. A mechanism for evading the local T cell immune response. J. Immunol. 155: 2240.
30. Suzuki, T., H. Tahara, S. Narula, K.W. Moore, P.D. Robbins, and M.T. Lotze. 1995. Viral interleukin 10 (IL-10), the human herpes virus 4 cellular IL-10 homologue, induces local anergy to allogenic and syngenic tumors. J. Exp. Med. 182: 447.
31. Fortis, C., M. Foppoli, L. Gianotti, L. Galli, G. Citterio, G. Consogno, O. Gentilini and M. Braga. 1196. Increased interleukin-10 serum levels in patients with solid tumours. Cancer Lett. 104: 1.
32. Murray, P.J., L. Wang, R.C. Onufry, R.I. Tepper,and R.A. Young. 1997. T cell-derived IL-10 antagononizes macrophage function in mycobacterial infection. J. Immunol. 158: 315.
33. Lehmann, A.K., A. Halstenen, S. Sornes, O. Rokkeand A. Waage. 1995. High levels of interleukin 10 in serum are associated with fatality in meningococcal disease. Infect. Immun. 63: 2109.
34. Clerici, M., T.A. Wynn, J.A. Berzofsky, R.L. Coffman, A. Sher, G.M. Shearer. 1994. Role of Interleukin-10 (IL-10) in T Helper Cell Dysfunction in Asymptomatic Individuals Infected with the Human Immunodeficiency Virus (HIV-1). J. Clin. Invest. 93:768.
35. VanFurth, A.M., E.M. Seijmonsbergen, J.A.M. Langermans, P.H.P. Groeneveld, C.E. Debel and R. VanFurth. 1995. High levels of interleukin 10 and tumor necrosis factor alpha in cerebrospinal fluid during the onset of bacterial meningite. Clin. Infect. Dis. 21: 220.
36. Llorente, L., Y. Richaud-Patin, J. Couderc, D. Alarcon-Segovia, R. Ruiz-Soto, N. Alcocer-Castillejos, J. Alcocer-Varela, J. Granados, S. Bahena, P. Galanaud and D. Emilia. 1997. Dysregulation of interleukin-10 production in relatives of patients with systemic lupus erythematosus. Arthritis Rheum. 38: 1429.
37. Westendorp, R.G.J., J.A.M. Langermans, T.W.G. Huizinga, A.H. Elouali, C.L. Verwej and J.P. Vandenbroucke. 1997. Genetic influence on cytokine production and fatal meningococcus disease. Lancet. 349:170.
38. Eskdale, J., G. gallagher, C.L. Vermeij, V. Keijsers, R.G.J. Westendorp and T.W.J. Huizinga. 1998. Interleukin 10 secretion in relation to human IL-10 locus haplotypes. Proc. Natl. Acad. Sci. USA. 95: 9465-9470.
39. Derkx, B., A. marchant, M. Goldman, R. Biilmer and S. Van de Venter. 1995. High levels of interleukin-10 during the initial phase of fulminant meningococcal septic shock. J. Infect. Dis. 171: 229.
40. Llorente, L., Y. Richaud-Patin, R. Fior, J. Alcocer-Varela, J. Wijdnes, B. Morel-Fourrier, P. Galanaud and P. Emilie. 1994. In vivo production of interleukin-10 by non -T cells in rheumatoid arthritis, Sjogren's syndrome, and systemic lupus erythematosus. A potential.mechanism of B lymphocytes hyperactivity and autoimmunity. Arthritis Rheum. 37: 1647.
41. Cash, J.J., J.B. Splawski, R. Thomas, J.F. McFarlin, H. Schulze-Koops, L.S. Davis, K. Fujita and P.E. Lipsky. 1995. Elevated interleukin-10 levels in patients with rheumatoid arthritis. Arthritis Rheum. 38: 96.
42. Eskdale, J. P. Wordsworth, S. Bowman, M. Field and G. Gallagher. 1997. Association between polymorphisms at the human IL-10 locus and systemic lupus erythematosus. Tissue Antigens. 49: 635.
43. Zheng, C., D. Huang, L. Liu, R. Wu, S. Bergenbrant Glas, A. Ostenborg, M. Bjorkholm, G. Holm, Q. Yi, A. Sundblad. 2001. Interleukin-10 gene promoter polymorphisms in multiple myeloma. Int. J. Cancer. 95: 184.
44. Via, C.S., G.C.Tsokos, N.I. Stocks, M. Clerici and G.M. Shearer. 1990. Human in vitro allogeneic responses: demonstration of three pathways of T helper cell activation. J. Immunol. 144:2524.
45. Hahn, A.B., J.C. kasten-Jolly, D. M. Costantino, E. Graffunder, T.P. Singh, G.K. Shen and D.J. Conti. 2001. Tnf-a, II-6, IFN-g, and IL-10 gene expression polymorphisms and the IL-4 receptor a-chain variant Q576R: effects on renal allograft outcome. Transplantation.. 72: 660.
46. Lio, D., G. Candore, A. Colombo, G. Colonna Romano, F. Gervasi, V. Marino, L. Scola and C. Caruso. 2001. A genetically determined high setting of TNF-alpha influences immunologic parameters of HLA-B8, DR3 positive subjects: implications for autoimmunity. Hum Immunol. 62: 705.
47. Akdis, C.A., and K. Blaser K. 2001. Mechanisms of interleukin-10-mediated immune suppression. Immunol. 103:131.
48. Segal, B.M., B.K. Dwyer and E.M. Shevach. 1998. An interleukin (IL)-10/IL-12 immunoregulatory circuit controls susceptibility to autoimmune disease J Exp Med 187: 537.
49. Bagli M, Papassotiropuolos A, Knapp M, Jessen F, Rao ML, Maier W, Heun R. association between an interleukin-6 and 3' flanking region haplotype and reduced Alzheimer's risk in German population. Neurosci Lett 2000; 283: 109-112.
50. Bowcock AM, Kidd JR, Lathrop GM, Daneshvar L, May LT, ray A, Sehgal PB, Kidd KK, Cvalli-Sforza LL. The human "interferon-beta 2/hepatocyte stimulating factor/IL-6" gene: DNA polymorphism studies and localization to chromosome 7p21. Genomics 1988; 3(1): 8-16.
51. Campbell IL, Stalder AK, Chiang CS, Bellinger R, Heyser CJ, Steffensen S, Masliah E, Powell HC, Gold LH, henriksen SJ, Siggins GR. Transgenic models to assess the pathogenic actions of cytokines in the central nervous system. Mol Psychiatry 1997; 2: 125-129.
52. Chao CC, Molitor TW, Hu S. Neuroprotective role of IL-4 against activated microglia. J Immunol 1993; 151: 1473-1481.
53. Falcone E, Spadafora P, De Luca M, Ruffolo R, Brancati C, De Benedictis G. DYS19, D12S67, and D1S80 polymorphisms in population sample from southern Italy and Greece. Hum Biol 1995; 67(5): 689-701
54. Fishman D, Faulds G, Jeffery R, Mohamed-Ali V, Yudkin JS, Humphries S, Woo P. The effect of novel polymorphisms in the interleukin-6 (IL-6) gene on IL-6 transcription and plasma IL-6 levels, and an association with systemic-onset juvenile chronic arthritis. J Clin Invest 1998; 102 (7): 1369-76.
55. Goris A, Epplen C, Fiten P, Andersson M, Murru R, sciacca FL, Ronsse I; jackel S, Epplen JT, Marrosu MG, Olsson T, Grimaldi LM, Opdenakker G, Billiau A, Vandenbroeck K. Analysis of IFN-gamma gene (IFNG) polymorphism in multiple sclerosis in Europe: effect of population structure on association with disease. J Interferon Cytokine Res 1999; 19(9): 1037- 1046.
56. Kilpinen S, Hulkkonen J, Wang XY, Hurme M. The promoter polymorphism of the IL-6 gene regulates IL-6 production in neonates but not in adults. Eur Cytokine Netw 2001; 12 (1): 62-8.
57. Kim JM, Brannan Cl, Copeland NG, Jenkins NA, Khan TA, Moore KW. Structure of the mouse IL-10 gene and chromosomal localisation of the mouse and human genes. Journal of Immunology 1992; 148: 3618.
58. Lio D, Scola L, Crivello A, Colonna-Romano G, Candore G, Bonafè M, Cavallone L, Franceschi C, Caruso C. Gender-specific association between -1082 IL-10 promoter polymorphism and longevity. Genes and Immunity 2002; 3: 30-33.
59. Llorente L, Richaud-Patin Y, Couderc J, Alarcon-Segovia D, Ruiz-Soto R, Alcocer-Castillejos N, Alcocer-Varela J, Granados J, Bahena S, Galanaud P, Emilia D. Dysregulation of interleukin-10 production in relatives of patients with systemic lupus erythematosus. Arthritis Rheum 1997;38(8): 1429-35.
60. Mateo I, Sanchez-Guerra M, Combarros O, Llorca J, Infante J, Gonzalez-Garcia J, del Molino JP, Berciano J. Lack of association between cathepsin D genetic polymorphism and Alzheimer disease in a Spanish sample. Am J Med Genet 2002; 114 (1): 31-3.
61. Murray RE, McGuigan F, Grant SF, Reid DM, Ralston SH. Polymorphisms of the interleukin-6 gene are associated with bone mineral density. Bone 1997; 21: 89-92.
62. Pallaud C, Stranieri C, Sass C, Siest G, Pignatti F, Visvikis S. Candidate gene polymorphimss in cardiovascular disease: a comparative study of frequencies between a French and an Italian population. Clin Chem Lab Med 2001; 39(2): 146-54.
63. Papassotiropoulos A, Hock C, Nitsch RM. Genetics of interleukin 6: implications for Alzheimer's disease. Neurobiology of Aging 1999;22: 863-871.
64. Pola R, Flex A, Gaetani E, Dal Lago A, Gerardini L, Pola P, Bernabei R. The -174 G/C polymorphism of the interleukin -6 gene promoter is associated with Alzheimer's disease in an Italian population. Neuroreport 2002; 13: 1645-1647.
65. Poli F, Nocco A, Berra S, Scalamogna M, Taioli E, Longhi , Sirchia G. Allele frequencies of polymorphisms of TNF-α, I-6, II-10 and IFNG in an Italian Caucasian population. European journal of Immunogenetics 2002; 29: 237-240.
66. Shibata N, Ohnuma T, Takahashi T, Baba H, Ishizuka T, Ohtsuka M, Ueki A, Nagao M, Arai H. Effect of IL-6 polymorphism on risk of Alzheimer disease: genotype-phenotype association study in Japanese cases. American Journal of Medical Genetics 2002; 114: 436-439.
67. Strauss S, Bauer J, Ganter U, Jonas U, Berger M, Volk B. detection of IL-6 and alpha 2-macroglobulin immunoreactivity in cortex and hippocampus of Alzheimer's disease patients. Lab Invest 1992; 66: 223-30.
68. Terry CF, Loukaci V, Green FR. Cooperative influence of genetic polymorphisms on interleukin 6 transcriptional regulation. J Biol Chem 2000; 275 (24): 18138-44.
69. Vandenabeele P, Fiers W.Is amyloidogenesis during Alzheimer's disease due to an IL-1/IL-6 mediated "acute phase response" in the brain?.Immunol Today 1991;12:217-19.
70. Vandenbroeck K, Hardt C, Louage j, Fiten P, Jackel S, Ronsse I, Epplen JT, Grimaldi LM, Olsson T, Marrosu MG, Billiau A, Opdenakker G. Lack of association between the interferon regulatory factor-1 (IRF1) locus at 5p31.1 and multiple sclerosis in Germany, northern Italy, Sardinia and Sweden. Genes Immun 2000; 1 (4): 290-2.
71. Quintana FJ. Carmi P. Cohen IR. DNA vaccination with heat shock protein 60 inhibits cyclophosphamide-accelerated diabetes. Journal of Immunology. 169(10):6030-5, 2002 Nov 15.
72. Dalpke AH. Opper S. Zimmermann S. Heeg K. Suppressors of cytokine signaling (SOCS)-1 and SOCS-3 are induced by CpG-DNA and modulate cytokine responses in APCs. Journal of Immunology. 166(12):7082-9, 2001 Jun 15.
73. Yi AK. Yoon JG. Yeo SJ. Hong SC. English BK. Krieg AM. Role of mitogen-activated protein kinases in CpG DNA-mediated IL-10 and IL-12 production: central role of extracellular signal-regulated kinase in the negative feedback loop of the CpG DNA-mediated Th1 response. Journal of Immunology. 168(9):4711-20, 2002 May 1.
74. G. Hartmann et al., "CpG DNA: A potent signal for growth, activation, and maturation of human dendritic cells," Proceedings of the National Academy of Sciences, 96:9305-10, 19975.Hollon T. Coley Toxin's Hidden message: from a 19th century mystery comes a potential new class of drugs: CpG oligonucleotides. The Scientist 15(5): 2001, March 5.
76. Hybridon's press release on May 07 2003 on data presented during the First Annual Meeting of the Federation of Clinical Immunology Societies held in Boston on May 4 to May 7, 2001, Posters#271"Modulation of immunostimulatory activity of CpG-oligonucleotides by incorporation of site-specific chemical modifications: Significance of internucleoside negative charge" Poster #229"Oligonucleotides containing YpG or CpR motifs as potent immunostimulatory agents", found on: hfttp://www.nrp-euro.com.
77. Du Y. et al. Association of an interleukin 1 alpha polymorphism with Alzheimer's disease. Neurology 55(4): 464-5 2000, Aug 22

## Claims

1. A method of determining the existence of or a predisposition to Alzheimer's disease, the method comprising analysing *in vitro* a DNA-bearing sample taken from a subject animal to determine the allelic variants present at one or more of the SNP loci at positions -1082, -819 and -592 of the gene encoding IL-10.

2. A method according to claim 1, in which the genotype at all three positions -1082, -819 and -592 is determined.

3. A method according to claim 1 or claim, 2 which further comprises analysing the sample to determine the alleles present for the genes encoding IL-6 and Apo-E.

4. A method according to claim 3 which further comprises analysing the sample to determine the alleles present for the gene encoding IL-1.

5. Use of the DNA or cDNA fragments encoding the IL-10 gene and comprising the allelic polymorphisms GCC/GCC, GCC/ACC, GCC/ATA, ACC/ACC, ACC/ATA, ATA/ATA in a method of screening compounds for their ability to modulate or prevent Alzheimer's disease.

## Patentansprüche

1. Verfahren zum Ermitteln des Vorliegens von der oder einer Veranlagung für die Alzheimer-Krankheit, wobei das Verfahren eine In-vitro-Analyse einer DNA-tragenden Probe von einem Probanden beinhaltet, um die an einem oder mehreren der SNP-Loci an den Positionen -1082, -819 und -592 des IL-10 kodierenden Gens vorhandenen Allelvarianten zu ermitteln.

2. Verfahren nach Anspruch 1, bei dem der Genotyp an allen drei Positionen -1082, -819 und -592 ermittelt wird.

3. Verfahren nach Anspruch 1 oder Anspruch 2, das ferner das Analysieren der Probe beinhaltet, um die Allele zu ermitteln, die für die IL-6 und Apo-E kodierenden Gene vorhanden sind.

4. Verfahren nach Anspruch 3, das ferner das Analysieren der Probe beinhaltet, um die für das IL-1 kodierende Gen vorhandenen Allele zu ermitteln.

5. Verwendung der DNA- oder cDNA-Fragmente, die das IL-10-Gen kodieren und die Allel-Polymorphismen GCC/GCC, GCC/ACC, GCC/ATA, ACC/ACC, ACC/ATA, ATA/ATA umfassen, in einem Verfahren zum Screenen von Verbindungen im Hinblick auf ihre Fähigkeit zur Modulation oder Verhütung der Alzheimer-Krankheit.

## Revendications

1. Procédé de détermination de l'existence de ou d'une prédisposition à la maladie d'Alzheimer, le procédé comprenant l'analyse in vitro d'un échantillon porteur d'ADN prélevé sur un animal sujet pour déterminer les variantes alléliques présentes en un ou plusieurs des locus SNP aux postions -1082, -819 et -592 du gène codant pour l'IL-10.

2. Procédé selon la revendication 1, dans lequel le génotype des trois positions -1082, -819 et -592 est déterminé.

3. Procédé selon la revendication 1 ou la revendication 2, qui comprend en outre l'analyse de l'échantillon pour déterminer les allèles présents pour les gènes codant pour l'IL-6 et Apo-E.

4. Procédé selon la revendication 3, qui comprend en outre l'analyse de l'échantillon pour déterminer les allèles présents pour le gène codant pour l'IL-1.

5. Utilisation des fragments d'ADN ou d'ADNc codant pour le gène d'IL-10 et comprenant les polymorphismes alléliques GCC/GCC, GCC/ACC, GCC/ATA, ACC/ACC, ACC/ATA, ATA/ATA dans un procédé de criblage de composés pour leur capacité à moduler ou empêcher la maladie d'Alzheimer.
